Europäisches Patentamt

European Patent Office

Office européen des brevets

(11) Veröffentlichungsnummer: **0 436 838 A1**

## (12) EUROPÄISCHE PATENTANMELDUNG

(21) Anmeldenummer: 90123421.1

(51) Int. Cl.5: **A61L 9/12, A63H 33/40**

(22) Anmeldetag: **06.12.90**

(30) Priorität: **12.12.89 DE 8914575 U**

(43) Veröffentlichungstag der Anmeldung:
**17.07.91 Patentblatt 91/29**

(84) Benannte Vertragsstaaten:
**AT BE CH DE DK ES FR GB GR IT LI LU NL SE**

(71) Anmelder: **Freytag von Loringhoven, Andreas**
**Mas St. Mathieu, Chemin Peyloubet**
**F-06130 Grasse(FR)**

(72) Erfinder: **Freytag von Loringhoven, Andreas**
**Mas St. Mathieu, Chemin Peyloubet**
**F-06130 Grasse(FR)**

(74) Vertreter: **Sturies, Herbert et al**
**Patentanwälte Dr. Ing. Dipl. Phys. Herbert**
**Sturies Dipl. Ing. Peter Eichler**
**Brahmsstrasse 29, Postfach 20 12 42**
**W-5600 Wuppertal 2(DE)**

(54) **Mobile.**

(57) Mobiles sind mit mehreren über Hängefäden (1) und waagebalkenartigen Querstäben (2) luftzugbeweglich aufzuhängenden, flachförmigen Figuren (3) versehen, die eine aus einem flachen gestanzten Zuschnitt bestehende, tragfähige Innenschicht und zu deren beiden Seiten damit verbundene Außenschichten aufweisen, die aus zu Darstellungen von Tieren, Blumen od.dgl. Bildern gewundenem textilem Fadenmaterial bestehen.

Um den Verwendungs- und Gebrauchszweck dieser gattungsgemäßen Mobiles noch wesentlich zu steigern, werden sie so ausgebildet, daß die Mobile-Figuren (3) eine Duftstoff-Innenschicht aus saugfähigem, mit raumodorierendem Duftstoff getränktem Material besitzen und einzeln für sich oder gemeinsam von einer leicht entfernbaren Plastik-Hülle (8) duftundurchlässig umschlossen sind.

Fig.1

EP 0 436 838 A1

bis 6 zeigen, mindestens eine aus einem flachen, insbesondere aus einem Stanzzuschnitt bestehende tragfähige Innenschicht 4 sowie zwei damit fest verbunden, z.B. aufgeklebte Außenschichten 5,6. Diese sind im vorliegenden Fall mit Darstellungen von Tieren versehen. Sie bestehen aus entsprechend gewundenem textilem Fadenmaterial.

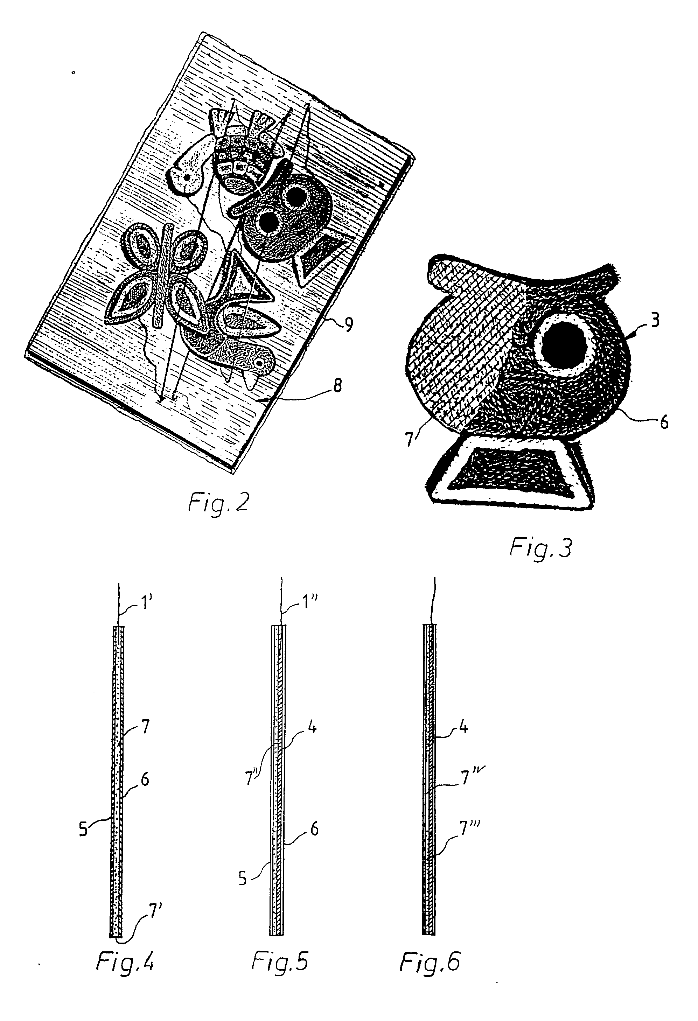

Die Mobile-Figuren 3 sind durchweg mit einer Innenschicht versehen, die aus saugfähigem, mit raumodorierendem Duftstoff getränktem Material besteht. Diese Duftstoffschicht 7 kann wie im Falle der Fig.4 zugleich die tragfähige Innenschicht bilden, in die der Hängefaden 1 eingebettet ist und die beidseitig mit den bebilderten Außenschichten 5, 6 versehen ist. Da diese Duftstoff-Innenschicht 7 beidseitig abgedeckt ist, steht für die Duftstoffabgabe an die Umgebungsluft unmittelbar nur deren Aussenrand 7 zur Verfügung, wodurch eine lang andauernde gesteuerte Duftstoffabgabe an die Umgebungsluft gewährleistet ist. Aber auch die aus textilem Fadengut bestehenden Außenschichten 5,6 reichern sich mit Duftstoff an und geben diesen zunehmend an die Außenluft ab, so daß sie mittelbar auch an der Duftstoffspeicherung und -abgabe beteiligt sind. Die Duftstoff-Innenschicht 7" kann auch wie im Falle der Fig.5 zusätzlich zur tragfähigen Innenschicht 4 mit dem an letzterer angreifenden Hängefaden 1" vorhanden sein, ggfs. sogar beidseitig von der mittleren Tragschicht 4. Im Falle der Fig.6 ist die Duftstoff-Innenschicht 7''' undurchgehend ausgebildet, das heißt mit dazwischen gelegenen Freistellen 7$^{IV}$ versehen, die hier auch als zusätzlicher Duftstoffspeicher dienen können.

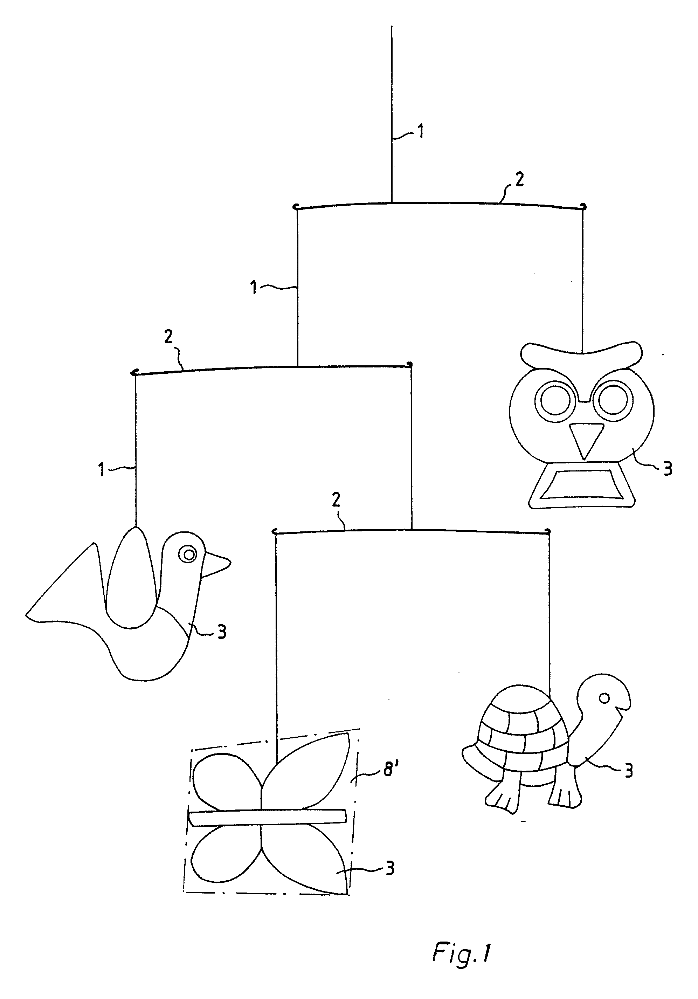

In allen Fällen werden die mit einem Duftstoff getränkten Mobiles für Aufbewahrungs- und Transportzwecke mit einer duftundurchlässigen Plastik-Hülle 8 umschlossen, damit eine vorzeitige Duftstoffabgabe unterbleibt. Es empfiehlt sich hierfür die in Fig.2 dargestellte Blisterpackung, bei der die einzelnen Teile des Mobiles in zusammengelegtem Zustand auf der aus Karton oder dergleichen bestehenden Bodenplatte 9 aufliegen und zusammen mit letzterer gemeinsam von der Plastik-Hülle 8 umschlossen sind. Stattdessen können die Mobile-Figuren auch einzeln in Plastik-Hüllen 8' duftundurchlässig umschlossen werden, wie das im Falle der Fig.1 links unten durch die strichpunktierte Umrahmung angedeutet ist.

## Patentansprüche

1. Mobile mit mehreren über Hängefäden (1) und waagebalkenartige Querstäbe (2) luftzugbeweglich aufzuhängenden, flachförmigen Figuren (3), die eine aus einem flachen gestanzten Zuschnitt bestehende, tragfähige Innenschicht (4) und zu deren beiden Seiten damit verbundene Außenschichten (5,6) aufweisen, die aus zu Darstellungen von Tieren, Blumen od.dgl. Bildern gewundenem textilem Fadenmaterial bestehen, **dadurch gekennzeichnet,** daß die Mobile-Figuren (3) eine Duftstoff-Innenschicht (7) aus saugfähigem, mit raumodorierendem Duftstoff getränktem Material besitzen und einzeln für sich oder gemeinsam von einer leicht entfernbaren Plastik-Hülle (8) duftundurchlässig umschlossen sind.

2. Mobile nach Anspruch 1, **dadurch gekennzeichnet,** daß die Duftstoff-Innenschicht (7) zugleich die tragfähige Innenschicht bildet (Fig.4).

3. Mobile nach Anspruch 1, **dadurch gekennzeichnet,** daß die Duftstoff-Innenschicht (7") neben der tragfähigen Innenschicht (4) vorhanden ist (Fig.5).

4. Mobile nach Anspruch 3, **dadurch gekennzeichnet,** daß beiderseits der tragfähigen Innenschicht (4) eine Duftstoff-Innenschicht vorhanden ist.

5. Mobile nach Anspruch 1, **dadurch gekennzeichnet,** daß die Duftstoff-Innenschicht (7''') undurchgehend verläuft (Fig.6).

6. Mobile nach einem der vorerwähnten Ansprüche 1 bis 5, **dadurch gekennzeichnet,** daß die Duftstoff-Innenschicht (7) aus einem mit Duftstoff besprühten Faservlies besteht.

7. Mobile nach einem der vorerwähnten Ansprüche 1 bis 6, **dadurch gekennzeichnet,** daß die Duftstoff-Innenschicht (7) aus mit Duftstoff besprühter Watte besteht.

8. Mobile nach einem der vorerwähnten Ansprüche 1 bis 5, **dadurch gekennzeichnet,** daß die Duftstoff-Innenschicht (7) aus mit Duftstoff besprühtem Textilstoff, z.B. aus Wolle besteht.

9. Mobile nach einem der vorerwähnten Ansprüche 1 bis 5, **dadurch gekennzeichnet,** daß die Duftstoff-Innenschicht (7) aus mit Duftstoff besprühtem Löschpapier besteht.

10. Mobile nach einem der vorerwähnten Ansprüche 1 bis 5, **dadurch gekennzeichnet,** daß die Duftstoff-Innenschicht (7) aus mit Duftstoff besprühtem bzw. getränktem, wabenartig hergestellten Kunststoffmaterial besteht.

*Fig.1*

EP 0 436 838 A1

Fig.2

Fig.3

Fig.4

Fig.5

Fig.6

5

<table>
<tr><td rowspan="2">Europäisches Patentamt</td><td rowspan="2">**EUROPÄISCHER RECHERCHENBERICHT**</td><td>Nummer der Anmeldung</td></tr>
<tr><td>**EP 90 12 3421**</td></tr>
</table>

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | US-A-4 419 395 (T. SUGIMOTO) <br> – – – | | A 61 L 9/12 <br> A 63 H 33/40 |
| A | US-A-4 145 001 (R.J. WEYENBERG) <br> – – – – – | | |

| | | | RECHERCHIERTE SACHGEBIETE (Int. Cl.5) |
|---|---|---|---|
| | | | A 61 L <br> A 63 H |

Der vorliegende Recherchenbericht wurde für alle Patentansprüche erstellt

| Recherchenort | Abschlußdatum der Recherche | Prüfer |
|---|---|---|
| Den Haag | 21 März 91 | PELTRE CHR. |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument
&: Mitglied der gleichen Patentfamilie, übereinstimmendes Dokument